# FASCICULE DE BREVET EUROPEEN

(11) **EP 3 616 192 B1**
(45) Date de publication et mention de la délivrance du brevet: **02.11.2022**
(21) Numéro de dépôt: 18725278.8
(22) Date de dépôt: 23.04.2018
(51) Int. Cl.: G10K 11/00, A61B 8/00, A61B 17/225, A61B 18/00, A61N 7/02

(54) **PROCEDE DE REFROIDISSEMENT D'UNE SONDE ULTRASONORE ET SONDE INCLUANT UN TEL REFROIDISSEMENT**
VERFAHREN ZUR KÜHLUNG EINER ULTRASCHALLSONDE UND SONDE MIT SOLCHER KÜHLUNG
METHOD FOR COOLING AN ULTRASONIC PROBE AND PROBE INCLUDING SUCH COOLING

(30) Priorité: 24.04.2017 FR 1753515
(43) Date de publication de la demande: 04.03.2020
(73) Titulaire: EDAP TMS France, 69120 Vaulx-en-Velin (FR)
(72) Inventeur: VINCENOT, Jérémy, 69100 Villeurbanne (FR); NALLET, Olivier, 69003 Lyon (FR); BLANC, Emmanuel, 69370 Saint Didier Au Mont D'or (FR)
(74) Mandataire: Cabinet Beau de Loménie
(86) Numéro de dépôt international: PCT/FR2018/051013
(87) Numéro de publication internationale: WO 2018/197795

(56) Documents cités:
- JP-A- 2004 097 402
- JP-A- 2007 144 225
- US-A- 6 126 619
- US-A1- 2011 011 111

## Description

La présente invention concerne le domaine technique des appareils ou des dispositifs comportant une sonde ultrasonore utilisée dans le traitement thérapeutique et pouvant éventuellement être associée avec une sonde ultrasonore d'imagerie échographique de l'anatomie humaine.

La présente invention vise plus précisément, la technique pour refroidir une sonde ultrasonore de thérapie voire également d'échographie.

L'objet de l'invention trouve une application particulièrement avantageuse dans le domaine des traitements thérapeutiques par des ondes ultrasonores focalisées à haute intensité (HIFU).

D'une manière générale, une sonde ultrasonore comporte un transducteur acoustique composé d'un matériau en particulier piézoélectrique qui convertit une énergie électrique en une énergie mécanique. En raison du rendement de conversion imparfait, l'énergie non convertie crée une accumulation de chaleur dans le matériau du transducteur. Un échauffement trop important peut entrainer un endommagement voire une destruction du transducteur.

L'état de la technique a proposé diverses solutions techniques pour dissiper la chaleur produite de manière à abaisser la température du transducteur.

Par exemple, le brevet EP 0 553 804 décrit un transducteur médical ultrasonore comportant un élément transducteur en contact avec un matériau conducteur de la chaleur pour assurer un refroidissement par conduction thermique vers l'arrière du transducteur. Ce mode de refroidissement, de type passif, c'est-à-dire sans circulation d'air ou de liquide est relativement limité et bien adapté à des transducteurs produisant peu de calories.

Il est également connu de faire appel à un système de refroidissement du type à conduction forcée utilisant un fluide de refroidissement mis en mouvement par une pompe. Par exemple, le brevet FR 2 910 169 décrit un système d'échographie comportant une sonde échographique et un système de refroidissement actif pour évacuer la chaleur de la sonde. Ce système de refroidissement comporte une pompe agencée pour faire circuler un agent de refroidissement dans la sonde échographique. Dans le même sens, le brevet US 5 560 362 décrit un transducteur ultrasonore pourvu d'un circuit de refroidissement comportant un échangeur de chaleur et une pompe assurant la circulation en boucle fermée, d'un fluide de refroidissement pour le transducteur. Dans les deux cas, le refroidissement du transducteur est obtenu par conduction thermique vers l'arrière du transducteur. L'inconvénient majeur de cette approche concerne la difficulté à incorporer, dans le même volume arrière, le dispositif de refroidissement et l'électronique de commande du transducteur.

Il est également connu de faire appel à un système de refroidissement du type à convection forcée utilisant un fluide de refroidissement mis en mouvement par une pompe. Une difficulté majeure d'un système de refroidissement à convection forcée consiste à obtenir un refroidissement homogène sur la totalité de la surface du transducteur. Cette difficulté est d'autant plus accentuée que la forme du transducteur s'éloigne d'une surface plane.

Dans le domaine des sondes ultrasonores de thérapie générant des puissances et des énergies acoustiques importantes, le brevet FR 2 929 040 décrit un transducteur constitué d'un assemblage de plusieurs éléments émetteurs, physiquement indépendants les uns et des autres. Ces éléments électro-acoustiques sont placés entre deux disques formant une chambre de refroidissement arrière. L'un des disques forme la face avant du transducteur qui contient le fluide de couplage acoustique enfermé dans une chambre avant fermée par une membrane. L'autre disque forme la face arrière qui reçoit l'électronique de commande.

Les éléments transducteurs sont formés chacun d'un élément piézoélectrique fixé sur une structure solide isolante électriquement et conductrice thermiquement. Cette structure solide permet ainsi le drainage de l'énergie thermique vers l'arrière du transducteur qui forme alors une sorte de radiateur lorsqu'il est exposé à un fluide de refroidissement.

Les éléments transducteurs sont répartis selon des spirales centrées sur la partie centrale du disque dans laquelle la sortie du fluide est aménagée, alors que les entrées du fluide sont aménagées à la périphérie du disque. Une telle disposition permet d'obtenir, en face arrière du transducteur, un écoulement homogène en spirale du fait d'une disposition en spirale des éléments transducteurs. Ce principe de refroidissement est spécifiquement adapté pour des éléments transducteurs indépendants pouvant former un canal de conduction du fluide de refroidissement en spirale. Un tel système de refroidissement ne s'avère pas adapté pour le refroidissement par la face avant d'un transducteur présentant une face d'émission lisse sur laquelle il n'est pas possible de placer des éléments qui permettraient de guider le liquide en vue d'obtenir un écoulement homogène car ces éléments viendraient interférer avec le champ ultrasonore. De façon complémentaire, la circulation du fluide de refroidissement en spirale guidée ne permet pas d'obtenir un refroidissement optimisé.

Il est également connu par le brevet US 6 126 619, un appareil transducteur médical à ultrasons conçu pour éliminer les bulles résiduelles contenues dans le fluide de refroidissement. Cet appareil comporte à cet effet, un transducteur concave avec une entrée du fluide en périphérie pour canaliser les bulles vers une évacuation centrale.

La demande de brevet JP 2004 097402 décrit un appareil de traitement par ondes ultrasonores comportant un système de circulation pour un fluide de refroidissement dans une enceinte de couplage du transducteur ultrasonore. L'entrée et la sortie du fluide de refroidissement sont situées dans la partie centrale du transducteur.

Le document JP 2007 144225 décrit un système thérapeutique ultrasonore extracorporel comportant un transducteur en forme de bande étroite en prévision d'un traitement intercostal. Ce système comporte également un dispositif de refroidissement par un liquide circulant à l'aide d'une pompe aspirante et d'une pompe refoulante pour maintenir un volume d'enveloppe de liquide de couplage constant. Les Figures 14 à 23 décrivent différentes variantes de réalisation du dispositif de refroidissement.

Les Figures 14, 16 et 17 décrivent des arrivées du liquide de refroidissement en périphérie du transducteur avec une déflexion du jet de liquide de refroidissement en direction du transducteur avec une évacuation par un orifice central. Une telle disposition assure une circulation centripète du liquide de refroidissement ne permettant pas un refroidissement optimisé.

Dans l'exemple de réalisation illustré à la Figure 19, la buse d'injection est positionnée pour diriger le liquide de refroidissement en direction du transducteur d'ultrasons selon une direction oblique. Le liquide de couplage coule ainsi en tournant à l'intérieur du générateur d'ultrasons et en montant jusqu'au point d'aspiration central. Une telle circulation centripète tournante

ne permet pas d'assurer un refroidissement optimisé du transducteur. Par ailleurs, il faut noter que la position centrale et non périphérique de la buse d'injection interfère avec le champ ultrasonore du transducteur nuisant à ses performances thérapeutiques.

La présente invention vise à remédier aux inconvénients de l'art antérieur en proposant une nouvelle technique de refroidissement pour une sonde ultrasonore permettant d'obtenir un refroidissement optimisé et homogène du transducteur quelle que soit la forme géométrique du transducteur.

Pour atteindre un tel objectif, l'invention concerne un procédé conforme à la revendication 1.

Selon l'invention le procédé consiste à créer entre l'entrée et la sortie, une circulation du fluide de refroidissement selon un tourbillon à l'intérieur de la chambre de refroidissement autour de l'axe de propagation acoustique de la face d'émission du transducteur.

Un autre objet de l'invention est de proposer une sonde de production d'ondes ultrasonores conforme à la revendication 2.

De plus, la sonde ultrasonore selon l'invention peut comporter en outre en combinaison au moins l'une et/ou l'autre des caractéristiques additionnelles suivantes :
- la sonde ultrasonore comporte au niveau de l'entrée, au moins une paroi de déflexion pour le fluide disposée en vis-à-vis de la sortie du conduit tubulaire pour orienter le fluide selon la direction tangentielle ;
- la face d'émission comporte un bord périphérique situé dans un plan dans lequel débouche par une sortie, le conduit tubulaire de guidage pour le fluide de refroidissement, une paroi de déflexion pour le fluide étant disposée en vis-à-vis de la sortie du conduit tubulaire ;
- le bord périphérique de la face d'émission est bordée à sa périphérie par un canal périphérique communiquant avec au moins un conduit tubulaire d'alimentation en fluide de refroidissement, ce canal étant pourvu d'au moins une d'ouverture centripète de communication avec la chambre de refroidissement ;
- la sonde ultrasonore comporte plusieurs ouvertures centripètes de communication délimitée chacune entre deux ailes voisines de déviation du fluide de refroidissement, avec un profil courbe variant d'une direction tangente à une direction quasi radiale ;
- la sonde ultrasonore comporte un conduit tubulaire d'alimentation en fluide de refroidissement débouchant dans la chambre de refroidissement et présentant une section droite de passage dont la normale est confondue à la direction tangentielle ;
- la chambre de refroidissement comporte une série d'entrées réparties à la périphérie de la face d'émission, en présentant chacune au moins un conduit tubulaire pour le fluide, orienté selon une direction pour créer ensemble une circulation tourbillonnaire du fluide de refroidissement à l'intérieur de la chambre de refroidissement ;
- la face d'émission présente une géométrie focalisante ;
- la face d'émission est tronquée de manière symétrique par rapport à un axe de propagation acoustique ;
- le transducteur comporte dans sa partie centrale, une deuxième sortie pour le fluide de refroidissement, les deux sorties étant de préférence, disposées symétriquement de part et d'autre de l'axe de propagation acoustique de la face d'émission ;
- le transducteur comporte dans sa partie centrale, une découpe délimitant un logement pour une sonde d'imagerie de part et d'autre de laquelle sont situées les sorties pour le fluide de refroidissement ;
- la sonde ultrasonore comporte une conduite d'alimentation en fluide débouchant par l'entrée dans la chambre de refroidissement, cette conduite comportant un dispositif de contrôle de la vitesse du fluide ;
- la sonde ultrasonore comporte un capteur de température adapté pour mesurer la température de la face arrière du transducteur, ce capteur de température étant relié au dispositif de contrôle de la vitesse du fluide permettant de contrôler la vitesse d'entrée du fluide dans la chambre de refroidissement en fonction de la mesure de température ;
- le conduit tubulaire possède une longueur supérieure ou égale soit à son diamètre pour un conduit de section circulaire, soit au plus petit diamètre pour un conduit de section oblongue ou soit à la plus petite largeur pour un conduit de section rectangulaire.

Diverses autres caractéristiques ressortent de la description faite ci-dessous en référence aux dessins annexés qui montrent, à titre d'exemples non limitatifs, des formes de réalisation de l'objet de l'invention.
La **Figure 1** est une vue en perspective montrant un exemple de réalisation d'une sonde ultrasonore de thérapie conforme à l'invention.
La **Figure 2** est une vue en partie arrachée de la sonde illustrée à la **Fig. 1****.**
La **Figure 3** est un schéma montrant le principe de refroidissement vortex d'une sonde conforme à l'invention.
La **Figure 4** est un schéma de principe d'une sonde conforme à l'invention montrant le refroidissement vortex sur une face d'émission concave.
La **Figure 5A** est un schéma de principe d'une sonde conforme à l'invention montrant le refroidissement vortex sur une face d'émission plane circulaire.
La **Figure 5B** est un schéma de principe d'une sonde conforme à l'invention montrant le refroidissement vortex sur une face d'émission plane non circulaire.
La **Figure 6** illustre un autre mode de réalisation d'une sonde conforme à l'invention montrant le refroidissement vortex à l'aide d'un déflecteur.
La **Figure 7** illustre un autre exemple de réalisation d'une chambre de refroidissement comportant plusieurs entrées.
La **Figure 8A** est une vue de détail d'un exemple de réalisation d'une entrée du fluide de refroidissement d'une sonde conforme à l'invention.
La **Figure 8B** illustre un détail de la conduite d'alimentation en fluide de refroidissement pour l'entrée illustrée à la **Fig.8A****.**
La **Figure 9** illustre un autre exemple de réalisation d'une chambre de refroidissement comportant plusieurs entrées.
La **Figure 10** est une vue en coupe prise sensiblement selon les lignes A-A de la **Fig. 9****.**

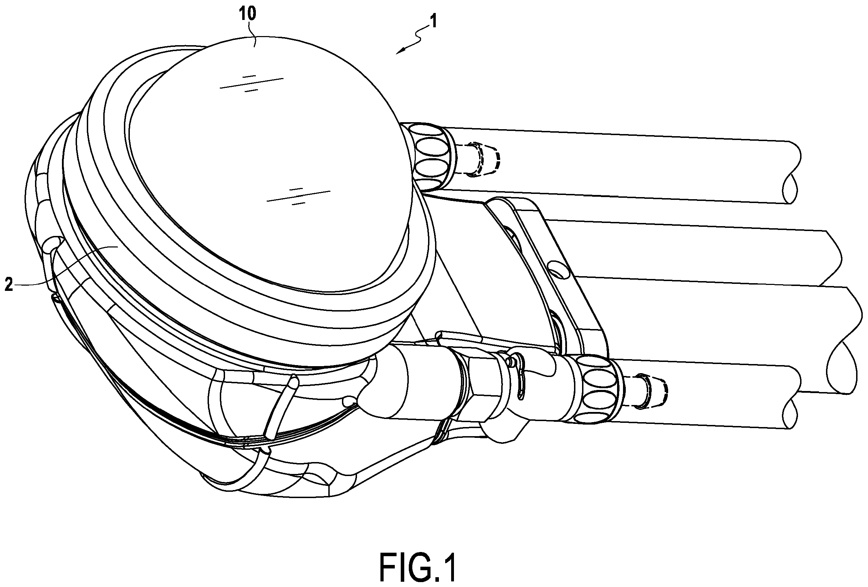

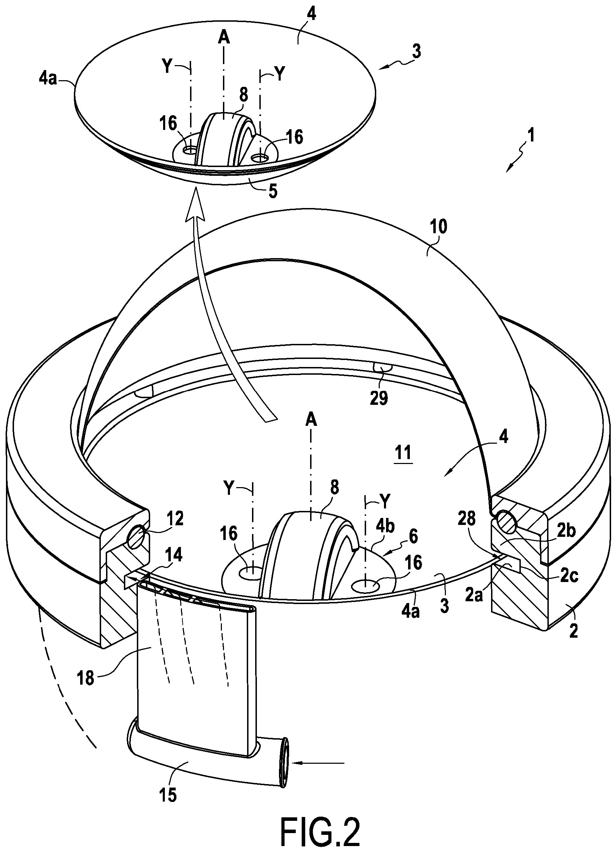

L'objet de l'invention concerne une nouvelle technique pour refroidir une sonde **1** de production d'ondes ultrasonores. Tel que cela ressort plus précisément des **Fig. 1** et **2****,** cette sonde **1** est utilisée plus particulièrement pour le traitement thérapeutique. Cette sonde de thérapie **1** fait partie d'un appareil de thérapie au sens général non représenté mais connu en soi et adapté pour réaliser le traitement de tissus d'un être vivant par l'intermédiaire d'ondes ultrasonores. Avantageusement, cette sonde de thérapie produit des ultrasons focalisés de haute intensité (HIFU). Bien entendu, la technique de refroidissement conforme à l'invention peut être mise en œuvre pour une sonde de production d'ondes ultrasonores de thérapie associée ou non avec une sonde d'imagerie échographique de l'anatomie humaine.

La sonde **1** comporte notamment un corps **2** de support pour un transducteur **3** comportant un ou plusieurs émetteurs ultrasonores tels que par exemple des éléments piézoélectriques. Les émetteurs ultrasonores du transducteur **3** sont reliés via, un étage amplificateur, à un circuit de commande délivrant des signaux pour activer les émetteurs ultrasonores. Le circuit de commande n'est pas décrit plus précisément car sa réalisation fait partie des connaissances techniques de l'homme du métier. Ce circuit de commande comporte ainsi classiquement un générateur de signal piloté qui est relié aux émetteurs ultrasonores par l'intermédiaire de l'étage amplificateur.

Le transducteur **3** présente à l'avant, une face d'émission **4** des ondes ultrasonores, et à l'arrière une face arrière **5.** Selon une variante de réalisation, un capteur de température peut éventuellement être présent sur cette face arrière afin de mesurer l'échauffement thermique au niveau de cette surface. La face d'émission **4** présente une surface lisse, en étant délimitée par un bord périphérique **4a** et éventuellement par un bord interne **4b** délimitant une découpe aménagée dans la partie centrale du transducteur.

De manière générale, la face d'émission **4** possède un axe de propagation des ondes acoustiques **A** correspondant à l'axe de symétrie et qui sera désigné dans la suite de la description par un axe de propagation acoustique **A.** Selon une variante avantageuse de réalisation, la face d'émission **4** présente une géométrie focalisante c'est à dire que les ondes ultrasonores produites sont focalisées dans une zone focale soit en raison du mode de commande des émetteurs ultrasonores soit par la forme géométrique de la face d'émission. Typiquement, la face d'émission **4** a une forme concave telle une forme hémisphérique et dans l'exemple illustré à la **Fig. 2****,** une forme torique. Avantageusement, le bord interne **4b** délimite un logement **6** pour notamment une sonde d'imagerie **8.** Selon cet exemple de réalisation, l'axe de propagation acoustique **A** correspond à l'axe de symétrie du tore formant la face d'émission **4** et se trouve être perpendiculaire au plan passant par le bord périphérique **4a** de la face d'émission.

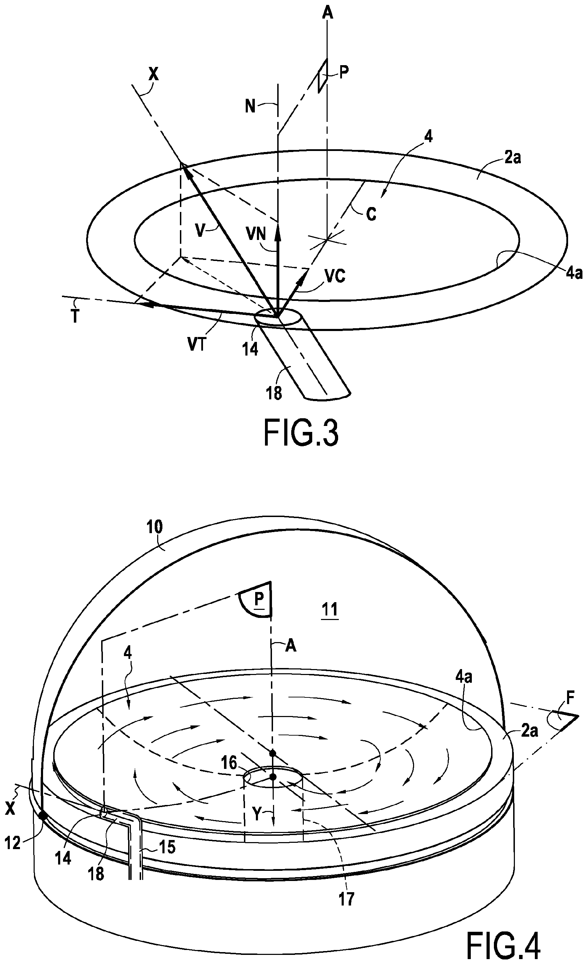

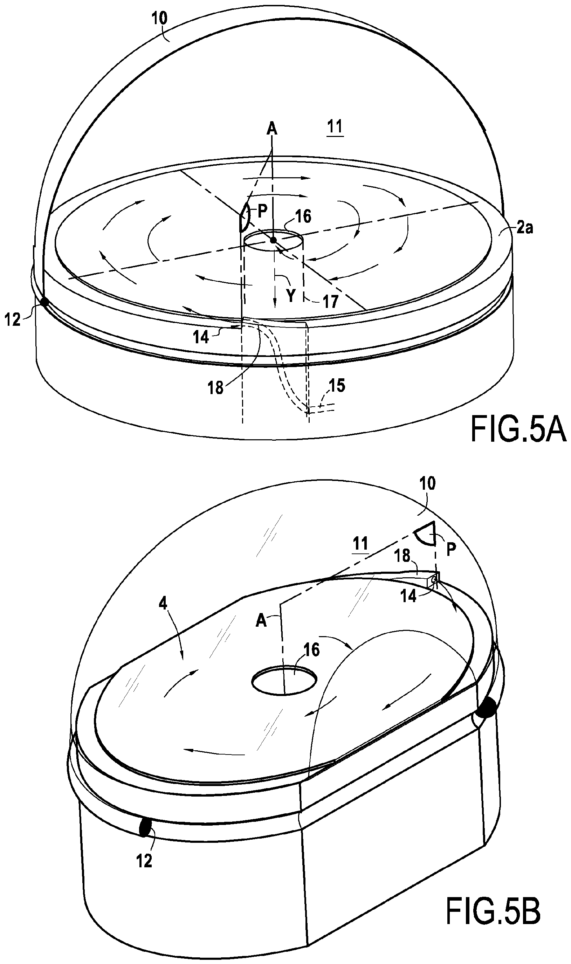

Bien entendu, la face d'émission **4** peut présenter une forme différente d'une forme concave telle qu'illustrée aux **Fig. 2** et **4****.** Dans les exemples illustrés aux **Fig. 5A, 5B****,** **6, 7** et **9****,** la face d'émission **4** présente une forme plane. Selon ces exemples de réalisation, l'axe de propagation acoustique **A** est perpendiculaire à la face d'émission **4.**

Selon les exemples de réalisation illustrés par les **Fig. 2****,** **4****,** **5A****,** **6****,** **7** et **9****,** la face d'émission **4** présente un contour de forme circulaire mais il est clair que la face d'émission peut présenter un contour de forme différente comme par exemple rectangulaire ou oblongue. Dans l'exemple illustré à la **Fig. 5B****,** la face d'émission **4** est tronquée de manière symétrique par rapport à l'axe de propagation acoustique **A** de sorte que la face d'émission **4** présente deux bords rectilignes s'étendant parallèlement en vis-à-vis et raccordés entre eux par deux bords circulaires de même rayon de courbure.

La sonde **1** comporte également une membrane **10** placée devant la face d'émission **4** et réalisée en un matériau transparent aux ondes ultrasonores. Cette membrane **10** délimite avec la face d'émission **4,** une chambre de refroidissement **11** dans laquelle circule un fluide de refroidissement. Cette membrane **10** est fixée sur le corps **2** par tous moyens appropriés avec un joint torique **12** pour obtenir une chambre de refroidissement étanche. Cette chambre de refroidissement **11** est également apte à remplir la fonction de couplage acoustique avec le milieu insonifié. Typiquement, le fluide de refroidissement est un liquide à base d'eau préalablement dégazée pour améliorer la propagation des ondes ou d'huile sélectionnée parmi celles présentant des caractéristiques acoustiques de faible absorption des ondes ultrasonores. On peut également utiliser le liquide décrit dans le brevet EP 1 038 551.

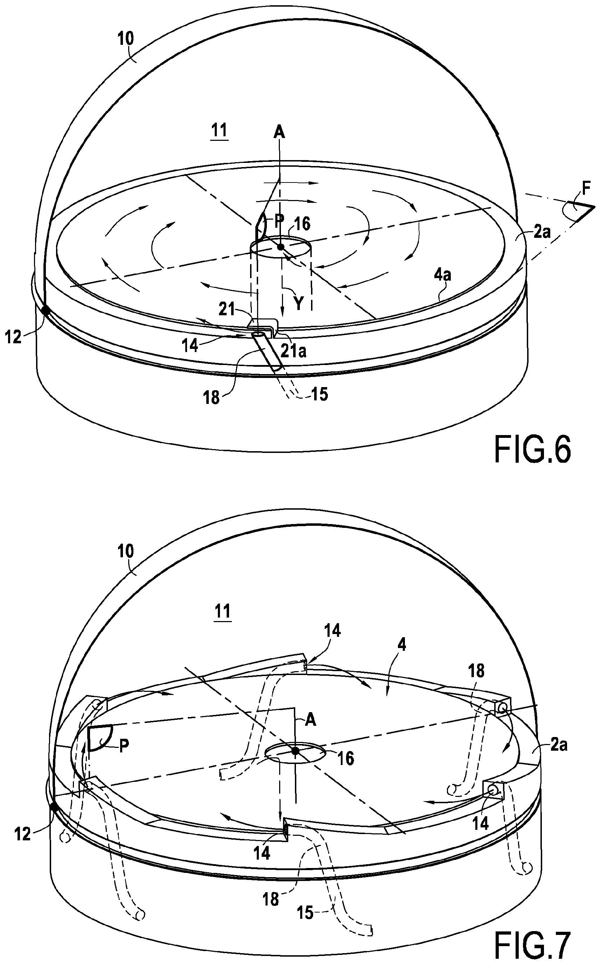

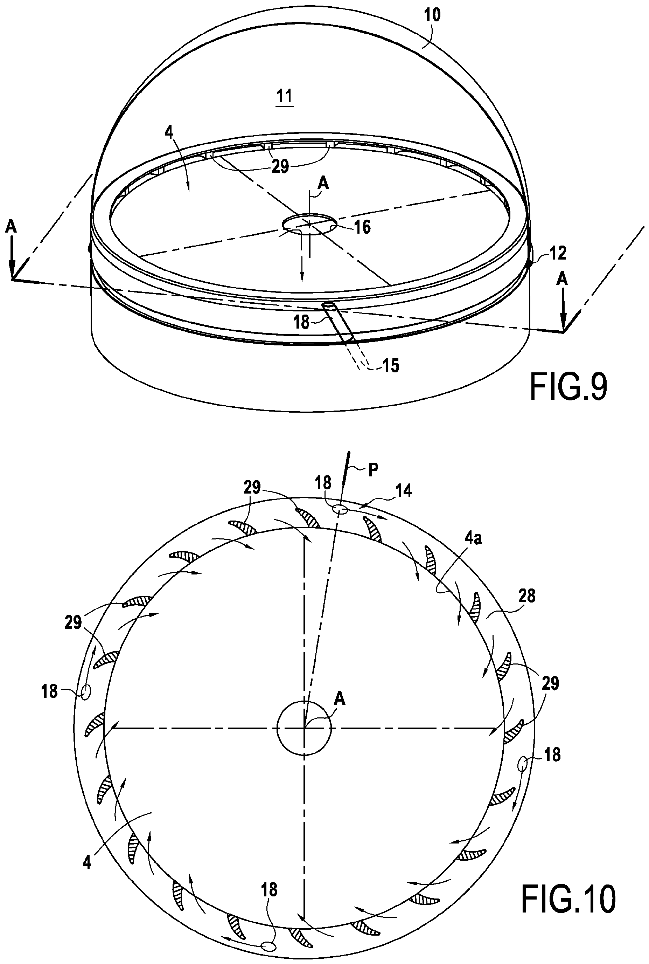

La chambre de refroidissement **11** comporte au moins une entrée **14** pour le fluide de refroidissement qui est amené par au moins une canalisation d'alimentation **15.** Chaque entrée **14** est située à la périphérie de la face d'émission **4** en dehors de la face d'émission pour ne pas perturber la propagation des ondes ultrasonores. Selon une caractéristique avantageuse de réalisation, chaque entrée **14** est réalisée dans une couronne **2a** du corps **2** entourant le transducteur au niveau du bord périphérique **4a** de la face d'émission. Chaque entrée **14** est donc située en dehors de la face d'émission **4,** en bordure du bord périphérique **4a** de la face d'émission. Dans les exemples de réalisation illustrés aux **Fig. 2****,** **4****,** **5A, 5B****,** **6****,** la chambre de refroidissement **11** comporte une unique entrée **14** alors que dans les exemples illustrés aux **Fig. 7** et **9****,** la sonde **1** comporte plusieurs entrées **14.**

La chambre de refroidissement **11** comporte également au moins une sortie **16** située dans la partie centrale de ladite face d'émission **4.** Chaque sortie **16** comporte une conduite de sortie **17** délimitant par son orifice d'entrée, une section de passage pour le fluide de refroidissement sortant de la chambre de refroidissement. Chaque sortie **16** présente un axe de sortie **Y** perpendiculaire à la section droite de passage de la conduite de sortie **17** récupérant le fluide sortant de la chambre de refroidissement **11.** Ainsi, la conduite de sortie **17** présente par son orifice, une section de passage qui permet l'évacuation du flux de liquide de refroidissement selon la direction **Y.** La section de passage pour la sortie **16** peut être de toutes formes possibles. La sonde **1** comporte une unique sortie **16** dans les exemples illustrés, à l'exception de l'exemple préféré illustré à la **Fig. 2** dans lequel la sonde comporte deux sorties **16** situées symétriquement de part et d'autre de l'axe de propagation acoustique **A** de la face d'émission.

Conformément à l'invention, la sonde **1** comporte au niveau de l'entrée **14,** au moins un conduit tubulaire de guidage **18** pour le fluide de refroidissement, communiquant avec la canalisation d'alimentation **15.** Ce conduit tubulaire **18** est adaptée pour créer à l'intérieur de la chambre de refroidissement **11,** entre l'entrée **14** et la sortie **16,** une circulation tourbillonnaire ou vortex du fluide de refroidissement autour de l'axe de propagation acoustique **A.** Cette circulation tourbillonnaire ou vortex conduit à un écoulement du fluide de refroidissement qui effectue au moins un tour de rotation autour de l'axe de propagation acoustique **A.** Chaque particule fluide décrit ainsi un mouvement de rotation supérieure à 360° autour de l'axe de propagation acoustique **A.** Les termes tourbillonnaire et vortex seront employés indifféremment dans la suite de la description pour décrire le phénomène de circulation du fluide de refroidissement en relation de la face d'émission du transducteur.

La circulation tourbillonnaire du fluide de refroidissement est réalisée autour d'un axe de rotation confondu avec l'axe de propagation acoustique **A** de la face d'émission. Tel est le cas notamment lorsque l'axe de sortie **Y** de la sortie **16** est confondu avec l'axe de propagation acoustique **A** de la face d'émission.

Il doit être noté que ce conduit tubulaire **18** de guidage pour le fluide, située au niveau de l'entrée **14** permet de définir par son extrémité débouchant dans la chambre de refroidissement **11,** une section droite de passage d'entrée pour le fluide de refroidissement. Ce conduit tubulaire **18** s'étend selon un axe longitudinal d'extension **X** en présentant une longueur déterminée suffisante adaptée pour canaliser l'écoulement du fluide de refroidissement et lui donner une vitesse dont le vecteur vitesse **V** est colinéaire à l'axe longitudinal d'extension **X,** c'est-à-dire perpendiculaire à la section droite de passage d'entrée. Tel que cela ressort plus précisément de la **Fig. 3****,** ce conduit tubulaire **18** est orienté de manière que son axe longitudinal d'extension **X** et par suite le vecteur vitesse qu'il porte, présente une composante tangentielle **VT** non nulle s'établissant selon une direction tangentielle **T** pour créer une circulation tourbillonnaire du fluide de refroidissement à l'intérieur de la chambre de refroidissement **11.**

Il est à noter que la direction tangentielle **T** est considérée par rapport au bord périphérique **4a** de la face d'émission **4,** cette direction tangentielle étant l'une des trois directions orthogonales d'un repère comportant une direction normale **N** orthogonale au plan contenant le bord périphérique **4a** de la face d'émission **4,** et une direction centripète **C** orthogonale à la direction tangentielle **T** et dirigée vers le centre de la face d'émission. Cette direction tangentielle **T** est sensiblement orthogonale à un plan **P** contenant l'axe de propagation acoustique **A** et au moins un point de l'entrée **14** c'est-à-dire par exemple le point d'intersection de l'axe longitudinal d'extension **X** avec la section droite de passage d'entrée définit par le conduit tubulaire **18.**

Comme indiqué ci-dessus, le conduit tubulaire **18** est orienté de manière que son axe longitudinal d'extension **X** et par suite le vecteur vitesse qu'il porte présente une composante tangentielle **VT** non nulle s'établissant selon une direction tangentielle **T.** D'une manière générale, ce vecteur vitesse **V** se décompose comme illustré à la **Fig. 3****,** selon les trois directions du repère à savoir, une direction tangentielle **T,** une direction centripète **C** et une direction normale **N,** en trois composantes respectivement, une composante tangentielle **VT,** une composante centripète **VC** et une composante normale **VN.**

Ce conduit tubulaire **18** possède à partir de son extrémité, une longueur déterminée selon l'axe longitudinal d'extension **X** pour canaliser le fluide de refroidissement et pour lui donner une orientation et une vitesse adaptées pour obtenir la circulation tourbillonnaire du fluide de refroidissement. Ainsi, le fluide de refroidissement présente au niveau de la section droite de passage d'entrée, un vecteur vitesse **V** avec une orientation selon l'axe longitudinal d'extension **X** et dont la composante tangentielle **VT** est non nulle.

Il ressort de la description qui précède que le conduit tubulaire **18** est agencé pour optimiser la composante tangentielle **VT** par rapport aux autres composantes. Idéalement, le conduit tubulaire **18** est agencé de manière que les composantes centripète **VC** et normale **VN** soient nulles ou tendent vers des valeurs nulles.

Ainsi, le conduit tubulaire **18** est orienté de manière que son axe longitudinal d'extension **X** et le vecteur vitesse qu'il porte présente une composante tangentielle telle que le ratio de la composante tangentielle du vecteur vitesse sur la composante centripète du vecteur vitesse est supérieur à 1.

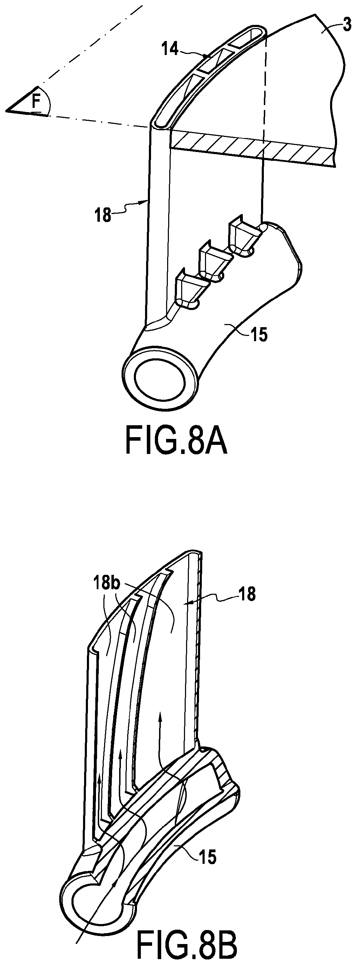

Idéalement, la longueur du conduit tubulaire **18** doit être supérieure ou égale à son diamètre pour un conduit tubulaire de section circulaire. A titre d'exemple, le conduit tubulaire **18** de la **Fig. 2** est circulaire et présente une longueur de 20 mm et un diamètre moyen de 2 mm alors que celui de la **Fig. 4** présente une longueur de 3 mm et un diamètre de 1 mm. Si le conduit tubulaire présente une section non circulaire mais oblongue ou rectangulaire telle que celle représentée aux **Fig. 8A** et **8B****,** la longueur du conduit tubulaire doit être supérieure ou égale au plus petit diamètre de la section oblongue ou supérieure ou égale à la plus petite largeur de la section rectangulaire. De préférence, la longueur du conduit tubulaire **18** est supérieure à 1,5 fois son diamètre pour un conduit de section circulaire. De même, la longueur du conduit tubulaire **18** est supérieure à 1,5 fois le plus petit diamètre d'une section oblongue ou la plus petite largeur de la section rectangulaire.

Ainsi, il est créé une circulation vortex du fluide de refroidissement autour de l'axe de propagation acoustique **A,** entre l'entrée **14** située à la périphérie de la face d'émission **4** et la sortie **16** située dans la partie centrale de la face d'émission **4.** Le vecteur vitesse **V** du fluide de refroidissement présente au niveau de la section de passage de l'entrée **14,** une direction dont une composante **VT** est non nulle et sensiblement perpendiculaire au plan **P.** Ainsi, le fluide de refroidissement prend une direction tangentielle par rapport au bord **4a** de la face d'émission **4** pour créer une circulation tourbillonnaire du fluide de refroidissement à l'intérieur de la chambre de refroidissement **11.**

La mise en œuvre au niveau de l'entrée **14,** d'au moins un conduit tubulaire **18** permet de créer, en combinaison avec la sortie **16,** cette circulation vortex du fluide de refroidissement dans la chambre de refroidissement **11.** Il est à noter que la canalisation **15** d'alimentation en fluide de refroidissement comporte un dispositif de contrôle de la vitesse du fluide permettant de conférer au fluide de refroidissement à l'entrée dans la chambre de refroidissement, une vitesse suffisante et adaptée pour obtenir une circulation tourbillonnaire. Typiquement, une vitesse de fluide au niveau de l'entrée **14** est comprise entre 10⁻³ m/s et 1 m/s.

Selon une variante avantageuse de réalisation dans laquelle la sonde comporte un capteur de température adapté pour mesurer la température de la face arrière du transducteur, le capteur de température est relié au dispositif de contrôle de la vitesse du fluide permettant de contrôler la vitesse d'entrée du fluide dans la chambre de refroidissement en fonction de la mesure faite par le capteur de température.

Cette circulation tourbillonnaire est réalisée quelle que soit la forme de la face d'émission **4.** A titre d'exemple, la **Fig. 4** illustre la circulation tourbillonnaire du fluide de refroidissement pour une face d'émission **4** concave tandis que les **Fig. 5A, 5B****,** **6, 7** et **9** montrent la circulation tourbillonnaire pour une face d'émission **4** plane.

Il doit être compris que l'objet de l'invention permet d'obtenir un refroidissement optimisé et homogène sur toute la surface de la face d'émission **4.** Comme expliqué précédemment, la circulation tourbillonnaire ou vortex du fluide de refroidissement autour de l'axe de propagation acoustique **A** met en œuvre un tourbillon unique.

La circulation du fluide de refroidissement selon un tourbillon unique permet d'un point de vue macroscopique, de conserver un déplacement laminaire du fluide de refroidissement, assurant un refroidissement homogène sur toute la surface de la face d'émission **4.** Par ailleurs, d'un point de vue microscopique, la circulation sous la forme d'un tourbillon augmente l'efficacité de refroidissement qui serait obtenue par une circulation directe, non tourbillonnaire du fluide de refroidissement entre un point d'entrée **14** et un point de sortie **16.** Le tourbillon obtenu permet à chaque particule du fluide de refroidissement d'effectuer au moins un tour de rotation autour de l'axe de propagation acoustique **A.** D'une manière générale, chaque particule du fluide de refroidissement tourne entre l'entrée **14** et la sortie **16,** selon plusieurs tours autour de l'axe de propagation acoustique **A.** Le nombre de tours de la particule fluide et donc sa vitesse de déplacement seront d'autant plus important que le ratio de la composante de vitesse tangentielle sur la composante de vitesse centripète sera largement supérieur à 1. Ce ratio tend vers l'infini lorsque la composante de vitesse centripète est nulle comme illustré à la **Fig. 4****.**

Selon un premier mode de réalisation, le conduit de guidage **18** du fluide est réalisé par une partie de la canalisation **15** d'alimentation du fluide de refroidissement débouchant par l'entrée **14** dans la chambre de refroidissement **11.** Comme cela apparait plus précisément à la **Fig. 4****,** la canalisation d'alimentation **15** comporte en amont de l'entrée **14,** le conduit tubulaire **18** orienté tangentiellement par rapport au bord **4a** de la face d'émission **4.** Selon cet exemple, la canalisation d'alimentation **15** comporte une partie s'étendant sensiblement parallèlement à l'axe de propagation acoustique **A** en se terminant par une partie coudée à 90° formant le conduit tubulaire de guidage **18** pour le fluide de refroidissement. Cette partie coudée à 90° formant le conduit tubulaire **18** permet de délimiter à son extrémité, la section droite de passage d'entrée pour le fluide de refroidissement. La normale de cette section droite de passage d'entrée correspond à l'axe longitudinal d'extension **X** qui est orienté selon la direction tangentielle **T** sensiblement orthogonale au plan **P** contenant l'axe de propagation acoustique **A.** Compte tenu de cette disposition, le vecteur vitesse **V** du fluide de refroidissement se décompose essentiellement en une composante tangentielle **VT,** selon la direction tangentielle **T** par rapport au bord **4a** de la face d'émission **4.**

Bien entendu, la canalisation d'alimentation **15** peut être complétement orientée tangentiellement par rapport au bord **4a** de la face d'émission **4,** même si cette solution est plus encombrante car la canalisation d'alimentation **15** et le conduite de sortie **17** sont, dans ce cas, orthogonales entre elles.

Selon un autre exemple de réalisation illustré à la figure **5A****,** la canalisation d'alimentation **15** comporte en amont de l'entrée **14,** une partie **18** formant le conduit tubulaire et orientée en sortie, tangentiellement par rapport au bord **4a** de la face d'émission **4.** Selon cet exemple, la canalisation d'alimentation **15** comporte une partie s'étendant sensiblement perpendiculairement à l'axe de sortie en se prolongeant par une partie cintrée **18** formant le conduit tubulaire et définissant à son extrémité, la section de passage d'entrée dont la normale est orthogonale au plan **P.** Là encore, le vecteur vitesse **V** du fluide de refroidissement se décompose essentiellement en une composante tangentielle **VT,** selon la direction tangentielle **T** par rapport à la face d'émission **4.**

Dans les exemples de réalisation illustrés aux **Fig. 4****,** **5A****,** **5B** **et** **7****,** le conduit tubulaire **18** est situé au-delà du plan frontal **F** de la face d'émission **4,** ce plan frontal **F** passant par le bord périphérique **4a** de cette face d'émission. Selon ces exemples, la conduite d'alimentation **15** comporte dans sa partie terminale, le conduit tubulaire **18** qui est orienté pour présenter une section droite de passage perpendiculaire au plan frontal et située en avant de ce plan frontal de sorte à obtenir un écoulement du fluide de refroidissement avec une vitesse colinéaire avec la direction tangentielle **T.**

Selon un deuxième mode de réalisation dont le principe est illustré à la **Fig. 6****,** le conduit tubulaire **18** est situé en retrait ou au niveau du plan frontal **F** de la face d'émission **4,** passant par le bord périphérique **4a** de cette face d'émission. En d'autres termes, le conduit tubulaire **18** du fluide de refroidissement débouche dans la couronne **2a** du support située dans le plan frontal de la face d'émission de sorte que l'extrémité du conduit tubulaire **18** et le bord périphérique **4a** de la face d'émission sont situés au même niveau.

Selon ce deuxième mode de réalisation, le conduit tubulaire **18** est orienté de manière que son axe longitudinal d'extension **X** et par suite, le vecteur vitesse **V** présente une composante tangentielle non nulle s'établissant selon une direction tangentielle **T** pour créer une circulation tourbillonnaire du fluide de refroidissement à l'intérieur de la chambre de refroidissement **11.** Selon cette configuration, l'axe longitudinal d'extension **X** et par suite le vecteur vitesse du fluide présente également une composante normale s'établissant selon la direction normale **N.** Selon cet exemple, la sonde comporte au niveau de l'entrée, au moins une paroi de déflexion **21** pour le fluide disposée en vis-à-vis de la sortie du conduit tubulaire pour orienter le fluide selon la direction tangentielle **T.** L'amplitude de la composante normale du vecteur vitesse est ainsi limitée.

Cette paroi de déflexion ou déflecteur **21** est disposé dans la chambre de refroidissement **11** en vis-à-vis du conduit tubulaire **18** et de la couronne **2a** du support. Comme cela apparaît plus précisément à la **Fig. 6****,** ce déflecteur **21** constitue un obstacle à l'arrivée à l'intérieur de la chambre, du fluide de refroidissement amené par le conduit tubulaire **18.** En combinaison avec la couronne **2a** du support, ce déflecteur **21** force ainsi le fluide de refroidissement à prendre une direction tangentielle par rapport à la face d'émission. Ce déflecteur **21** comporte ainsi une partie frontale orientée selon la direction tangentielle **T** située en vis-à-vis de la sortie du conduit tubulaire **18.**

Selon une variante avantageuse de réalisation, le déflecteur **21** est prolongé à l'équerre par une partie d'arrêt **21a** permettant d'orienter le fluide de refroidissement dans un unique sens tangentiel.

La **Fig. 2** illustre une variante de réalisation de ce principe dans laquelle la face d'émission **4** comporte un bord périphérique **4a** circulaire qui est bordée à sa périphérie par un canal périphérique **28** communiquant avec un ou plusieurs conduits tubulaires **18** d'alimentation en fluide de refroidissement débouchant dans la couronne **2a.** Les conduits tubulaires **18** du fluide de refroidissement débouchent dans le bord périphérique **2a** du support situé dans le plan frontal de la face d'émission de sorte que l'extrémité des conduits tubulaires **18** et le bord périphérique **4a** de la face d'émission sont situés au même niveau.

Tel que cela ressort plus précisément des **Fig. 8A** à **8B****,** la canalisation d'alimentation **15** en fluide de refroidissement communique avec un conduit tubulaire **18** débouchant dans la couronne **2a.** Pour réduire les pertes de charge liées au conduit tubulaire **18** sans pour autant augmenter sa section, le conduit tubulaire **18** comporte avantageusement plusieurs couloirs d'acheminement **18b** parallèles, au nombre de trois dans l'exemple illustré, aménagés pour épouser la forme circulaire de la couronne **2a.** Ce conduit tubulaire **18** débouche ainsi selon un secteur limité de la circonférence du bord périphérique **4a** de la face d'émission.

Pour chaque entrée, le fluide de refroidissement est guidé selon la direction tangentielle, par le conduit tubulaire **18** mais également par une paroi de déflexion **2b** située en vis-à-vis et à distance de la section de sortie du conduit tubulaire **18.** Cette paroi de déflexion **2b** joue le rôle de déflecteur pour le fluide de refroidissement pour orienter le fluide selon la direction tangentielle de sorte que l'amplitude de la composante normale du vecteur vitesse est ainsi limitée.

Dans l'exemple illustré, la paroi de déflexion **2b** est réalisée sous la forme d'une bordure annulaire s'étendant en vis-à-vis de la couronne **2a** du support. La couronne **2a** du support est reliée à la bordure annulaire **2b** par l'intermédiaire d'une paroi de liaison **2c.** La bordure annulaire **2b** qui s'étend en vis-à-vis et à distance de la couronne **2a** délimite un canal **28** périphérique réalisé, comme expliqué ci-dessus, par une rainure aménagée dans le corps **2** de manière à être fermé vers l'extérieur de la chambre et communiquer intérieurement avec la partie centrale de la chambre de refroidissement.

Le canal périphérique **28** comporte des plots **29** ponctuels de liaison mécanique entre la couronne **2a** du support et la bordure annulaire **2b.** Dans l'exemple de réalisation illustré aux **Fig. 9** et **10****,** les plots **29** sont réalisés chacun sous la forme d'une aile de déviation du fluide de refroidissement. L'espace entre chaque plot forme ainsi une ouverture centripète donnant au fluide l'accès à la chambre de refroidissement **11.** Chaque plot **29** possède un profil courbe variant d'une direction tangente à une direction quasi radiale afin de limiter les pertes de charge.

Selon une variante de réalisation illustrée à la **Fig. 7****,** la chambre de refroidissement **11** comporte une série d'entrées **14** réparties à la périphérie de la face démission **4,** en présentant chacune au moins un conduit tubulaire **18** de guidage pour le fluide, orienté selon une direction orthogonale au plan **P** contenant l'axe de propagation acoustique **A,** pour créer ensemble une circulation tourbillonnaire du fluide de refroidissement à l'intérieur de la chambre de refroidissement. Bien entendu, toutes les variantes de la sonde selon l'invention peuvent être équipées de plusieurs entrées.

Dans les exemples illustrés aux **Fig. 4****,** **5A****,** **5B****,** **6****,** **7****,** et **9****,** la chambre de refroidissement **11** comporte une unique sortie **16** dont l'axe de sortie **Y** est confondu avec l'axe de propagation acoustique **A** de la face d'émission **4.** Selon la variante de réalisation illustrée à la **Fig. 2****,** le transducteur **3** comporte dans sa partie centrale, une deuxième sortie **16** pour le fluide de refroidissement. Les deux sorties **16** sont disposées symétriquement de part et d'autre de l'axe de propagation acoustique **A** de la face d'émission **4** avec un axe de sortie **Y** colinéaire avec l'axe de propagation acoustique **A** de la face d'émission **4.** Bien entendu, toutes les variantes de la sonde selon l'invention peuvent être équipées de plusieurs sorties. Selon cette variante, le fluide de refroidissement circule également selon un tourbillon unique tournant autour de l'axe de propagation acoustique **A.**

Il ressort de la description qui précède que l'objet de l'invention propose un nouveau procédé pour refroidir un transducteur d'une sonde de production d'ondes ultrasonores, consistant à créer entre l'entrée et la sortie de la chambre de refroidissement de la face d'émission, une circulation tourbillonnaire du fluide de refroidissement à l'intérieur de la chambre de refroidissement autour d'un axe perpendiculaire à la partie centrale du transducteur. Cette circulation vortex à savoir une circulation selon un tourbillon unique améliore à la fois la performance et l'homogénéité du refroidissement par conduction de la sonde ultrasonore de thérapie voire d'une sonde d'imagerie placée en son centre.

Bien entendu, l'objet de l'invention peut être mis en œuvre pour refroidir également la face arrière du transducteur.

L'invention n'est pas limitée aux exemples décrits et représentés car diverses modifications peuvent y être apportées sans sortir du cadre de l'invention tel que défini par les revendications annexées.

## Revendications

1. - Procédé pour refroidir un transducteur (**3**) d'une sonde de production d'ondes ultrasonores, le transducteur (**3**) présentant, à l'avant, une face d'émission (**4**) des ondes ultrasonores avec un axe de propagation acoustique (**A**) des ondes ultrasonores, et à l'arrière une face arrière (**5**), au moins la face d'émission (**3**) délimitant en partie une chambre de refroidissement (**11**) dans laquelle un fluide de refroidissement est amené par un conduit tubulaire (**18**) s'étendant en amont d'une entrée (**14**), avec une longueur déterminée selon un axe longitudinal d'extension (**X**) pour canaliser le fluide de refroidissement avec un vecteur vitesse, le fluide de refroidissement circulant entre au moins l'entrée (**14**) et au moins une sortie (**16**), l'entrée (**14**) étant située à la périphérie de la face d'émission (**4**) tandis que la sortie (**16**) est située dans la partie centrale de ladite face d'émission, **caractérisée en ce qu'**il consiste à orienter le conduit tubulaire (**18**) de manière que son axe longitudinal d'extension (**X**) et le vecteur vitesse qu'il porte présentent une composante tangentielle s'établissant selon une direction tangentielle sensiblement orthogonale à un plan (**P**) contenant l'axe de propagation acoustique (**A**) et l'entrée **(14)**, et telle que le ratio de la composante tangentielle du vecteur vitesse sur la composante centripète du vecteur vitesse est supérieur à 1, pour créer entre l'entrée (**14**) et la sortie (**16**), une circulation du fluide de refroidissement selon un tourbillon à l'intérieur de la chambre de refroidissement autour de l'axe de propagation acoustique (**A**) de la face d'émission du transducteur.

2. - Sonde de production d'ondes ultrasonores, comportant un transducteur (**3**) présentant, à l'avant, une face d'émission (**4**) des ondes ultrasonores avec un axe de propagation acoustique (**A**) des ondes ultrasonores, et à l'arrière une face arrière (**5**), au moins la face d'émission (**4**) délimitant en partie une chambre de refroidissement (**11**) dans laquelle un fluide de refroidissement est amené par un conduit tubulaire, à circuler entre au moins une entrée (**14**) située à la périphérie de la face d'émission (**4**) et au moins une sortie (**16**) située dans la partie centrale de ladite face d'émission, le conduit tubulaire s'étendant en amont de l'entrée, avec une longueur déterminée selon un axe longitudinal d'extension (**X**) pour canaliser le fluide de refroidissement avec un vecteur vitesse, **caractérisée en ce que** le conduit tubulaire (**18**) est orienté de manière que son axe longitudinal d'extension (**X**) et le vecteur vitesse qu'il porte présentent une composante tangentielle s'établissant selon une direction tangentielle sensiblement orthogonale à un plan (**P**) contenant l'axe de propagation acoustique (**A**) et l'entrée **(14)**, et telle que le ratio de la composante tangentielle du vecteur vitesse sur la composante centripète du vecteur vitesse est supérieur à 1, pour guider le fluide en vue de créer une circulation du fluide de refroidissement selon un tourbillon à l'intérieur de la chambre de refroidissement (**11**).

3. - Sonde ultrasonore selon la revendication 2, **caractérisée en ce qu'**elle comporte au niveau de l'entrée, au moins une paroi (**21, 2b**) de déflexion pour le fluide disposée en vis-à-vis de la sortie du conduit tubulaire pour orienter le fluide selon la direction tangentielle.

4. - Sonde ultrasonore selon la revendication 3, **caractérisée en ce que** la face d'émission (**4**) comporte un bord périphérique (**4a**) situé dans un plan dans lequel débouche par une sortie, le conduit tubulaire (**18**) de guidage pour le fluide de refroidissement, une paroi (**21, 2b**) de déflexion pour le fluide étant disposée en vis-à-vis de la sortie du conduit tubulaire.

5. - Sonde ultrasonore selon la revendication 4, **caractérisée en ce que** le bord périphérique (**4a**) de la face d'émission (**4**) est bordée à sa périphérie par un canal périphérique (**28**) communiquant avec au moins un conduit tubulaire d'alimentation (**18**) en fluide de refroidissement, ce canal (**28**) étant pourvu d'au moins une d'ouverture centripète de communication avec la chambre de refroidissement.

6. - Sonde ultrasonore selon la revendication 5, **caractérisée en ce qu'**elle comporte plusieurs ouvertures centripètes de communication délimitées chacune entre deux ailes (**29**) voisines de déviation du fluide de refroidissement, avec un profil courbe variant d'une direction tangente à une direction quasi radiale.

7. - Sonde ultrasonore selon la revendication 2, **caractérisée en ce que** la sonde (**1**) comporte un conduit tubulaire (**18**) d'alimentation en fluide de refroidissement débouchant dans la chambre de refroidissement (**11**) et présentant une section droite de passage dont la normale est confondue à la direction tangentielle.

8. - Sonde ultrasonore selon l'une des revendications 2 à 7, **caractérisée en ce que** la chambre de refroidissement (**11**) comporte une série d'entrées (**14**) réparties à la périphérie de la face d'émission, en présentant chacune au moins un conduit tubulaire (**18**) pour le fluide, orienté selon une direction pour créer ensemble une circulation tourbillonnaire du fluide de refroidissement à l'intérieur de la chambre de refroidissement (**11**).

9. - Sonde ultrasonore selon l'une des revendications 2 à 8, **caractérisée en ce que** la face d'émission (**4**) présente une géométrie focalisante.

10. - Sonde ultrasonore selon la revendication 9, **caractérisée en ce que** la face d'émission (**4**) est tronquée de manière symétrique par rapport à un axe de propagation acoustique.

11. - Sonde ultrasonore selon l'une des revendications 2 à 10, **caractérisée en ce que** le transducteur (**3**) comporte dans sa partie centrale, une deuxième sortie (**16**) pour le fluide de refroidissement, les deux sorties étant de préférence, disposées symétriquement de part et d'autre de l'axe de propagation acoustique de la face d'émission (**4**).

12. - Sonde ultrasonore selon la revendication 11, **caractérisée en ce que** le transducteur (**3**) comporte dans sa partie centrale, une découpe délimitant un logement (**6**) pour une sonde d'imagerie (**8**) de part et d'autre de laquelle sont situées les sorties (**16**) pour le fluide de refroidissement.

13. - Sonde ultrasonore selon l'une des revendications 2 à 12, **caractérisée en ce que** la sonde comporte une conduite (**15**) d'alimentation en fluide débouchant par l'entrée dans la chambre de refroidissement (**11**), cette conduite comportant un dispositif de contrôle de la vitesse du fluide.

14. - Sonde ultrasonore selon la revendication 13, **caractérisée en ce qu'**elle comporte un capteur de température adapté pour mesurer la température de la face arrière du transducteur (**3**), ce capteur de température étant relié au dispositif de contrôle de la vitesse du fluide permettant de contrôler la vitesse d'entrée du fluide dans la chambre de refroidissement en fonction de la mesure de température.

15. - Sonde ultrasonore selon l'une des revendications 2 à 14, **caractérisée en ce que** le conduit tubulaire (**18**) possède une longueur supérieure ou égale soit à son diamètre pour un conduit de section circulaire, soit au plus petit diamètre pour un conduit de section oblongue ou soit à la plus petite largeur pour un conduit de section rectangulaire.

## Patentansprüche

1. Verfahren zum Kühlen eines Wandlers (3) einer Sonde zur Erzeugung von Ultraschallwellen, wobei der Wandler (3) vorne eine Seite zum Senden (4) der Ultraschallwellen mit einer Schallausbreitungsachse (A) der Ultraschallwellen und hinten eine Rückseite (5) aufweist, wobei wenigstens die Sendeseite (3) teilweise eine Kühlkammer (11) begrenzt, in die ein Kühlfluid durch eine röhrenförmige Leitung (18) zugeführt wird, die sich stromaufwärts eines Eingangs (14) mit einer bestimmten Länge entlang einer Erstreckungslängsachse (X) erstreckt, um das Kühlfluid mit einem Geschwindigkeitsvektor zu leiten, wobei das Kühlfluid zwischen wenigstens einem Eingang (14) und wenigstens einem Ausgang (16) zirkuliert, wobei der Eingang (14) am Umfang der Sendeseite (4) angeordnet ist, während der Ausgang (16) im mittleren Teil der Sendeseite (4) angeordnet ist, **dadurch gekennzeichnet, dass** es darin besteht, die röhrenförmige Leitung (18) so auszurichten, dass ihre Erstreckungslängsachse (X) und der Geschwindigkeitsvektor, den sie trägt, eine tangentiale Komponente aufweisen, die entlang einer tangentialen Richtung gebildet wird, die im Wesentlichen orthogonal zu einer Ebene (P) ist, welche die Schallausbreitungsachse (A) und den Eingang (14) enthält, und derart, dass das Verhältnis der tangentialen Komponente des Geschwindigkeitsvektors zur zentripetalen Komponente des Geschwindigkeitsvektors größer als 1 ist, um zwischen dem Eingang (14) und dem Ausgang (16) eine Zirkulation des Kühlfluids in einem Wirbel innerhalb der Kühlkammer um die Schallausbreitungsachse (A) der Sendeseite des Wandlers herum zu erzeugen.

2. Sonde zur Erzeugung von Ultraschallwellen, umfassend einen Wandler (3), der vorne eine Seite zum Senden (4) der Ultraschallwellen mit einer Schallausbreitungsachse (A) der Ultraschallwellen und hinten eine Rückseite (5) aufweist, wobei wenigstens die Sendeseite (3) teilweise eine Kühlkammer (11) begrenzt, in der ein Kühlfluid durch eine röhrenförmige Leitung (18) dazu gebracht wird, zwischen wenigstens einem Eingang (14), der am Umfang der Sendeseite (4) angeordnet ist, und wenigstens einem Ausgang (16), der im mittleren Teil der Sendeseite (4) angeordnet ist, zu zirkulieren, wobei sich die röhrenförmige Leitung stromaufwärts des Eingangs, mit einer bestimmten Länge entlang einer Erstreckungslängsachse (X) erstreckt, um das Kühlfluid mit einem Geschwindigkeitsvektor zu leiten, **dadurch gekennzeichnet, dass** die röhrenförmige Leitung (18) derart ausgerichtet ist, dass ihre Erstreckungslängsachse (X) und der Geschwindigkeitsvektor, den sie trägt, eine tangentiale Komponente aufweisen, die entlang einer tangentialen Richtung gebildet wird, die im Wesentlichen orthogonal zu einer Ebene (P) ist, welche die Schallausbreitungsachse (A) und den Eingang (14) enthält, und derart, dass das Verhältnis der tangentialen Komponente des Geschwindigkeitsvektors zur zentripetalen Komponente des Geschwindigkeitsvektors größer als 1 ist, um das Fluid zum Erzeugen einer Zirkulation des Kühlfluids in einem Wirbel innerhalb der Kühlkammer zu leiten.

3. Ultraschallsonde nach Anspruch 2, **dadurch gekennzeichnet, dass** sie im Bereich des Eingangs wenigstens eine Wand (21, 2b) zum Ablenken für das Fluid aufweist, die gegenüber dem Ausgang der röhrenförmigen Leitung angeordnet ist, um das Fluid in der tangentialen Richtung auszurichten.

4. Ultraschallsonde nach Anspruch 3, **dadurch gekennzeichnet, dass** die Sendeseite (4) einen Umfangsrand (4a) aufweist, der in einer Ebene liegt, in der die röhrenförmige Leitung (18) zum Leiten für das Kühlfluid über einen Ausgang ausmündet, wobei eine Wand (21, 2b) zum Ablenken für das Fluid gegenüber dem Ausgang der röhrenförmigen Leitung angeordnet ist.

5. Ultraschallsonde nach Anspruch 4, **dadurch gekennzeichnet, dass** der Umfangsrand (4a) der Sendeseite (4) an seinem Umfang durch einen Umfangskanal (28) eingefasst ist, der mit wenigstens einer röhrenförmigen Leitung zum Zuführen (18) von Kühlfluid in Verbindung steht, wobei dieser Kanal (28) mit wenigstens einer zentripetalen Öffnung zur Verbindung mit der Kühlkammer versehen ist.

6. Ultraschallsonde nach Anspruch 5, **dadurch gekennzeichnet, dass** sie mehrere zentripetale Verbindungsöffnungen umfasst, die jeweils zwischen zwei benachbarten Flügeln (29) zum Ablenken des Kühlfluids begrenzt sind, mit einem gekrümmten Profil, das von einer tangentialen Richtung zu einer quasi radialen Richtung variiert.

7. Ultraschallsonde nach Anspruch 2, **dadurch gekennzeichnet, dass** die Sonde (1) eine röhrenförmige Leitung (18) zum Zuführen von Kühlfluid umfasst, welche in die Kühlkammer (11) mündet und einen Durchgangsquerschnitt aufweist, dessen Normale mit der tangentialen Richtung zusammenfällt.

8. Ultraschallsonde nach einem der Ansprüche 2 bis 7, **dadurch gekennzeichnet, dass** die Kühlkammer (11) eine Reihe von Eingängen (14) aufweist, die am Umfang der Sendeseite verteilt sind und dabei jeweils wenigstens eine röhrenförmige Leitung (18) für das Fluid aufweisen, die in einer Richtung ausgerichtet ist, um zusammen eine Wirbelzirkulation des Kühlfluids innerhalb der Kühlkammer (11) zu erzeugen.

9. Ultraschallsonde nach einem der Ansprüche 2 bis 8, **dadurch gekennzeichnet, dass** die Sendeseite (4) eine fokussierende Geometrie aufweist.

10. Ultraschallsonde nach Anspruch 9, **dadurch gekennzeichnet, dass** die Sendeseite (4) in Bezug zu einer Schallausbreitungsachse symmetrisch abgeschnitten ist.

11. Ultraschallsonde nach einem der Ansprüche 2 bis 10, **dadurch gekennzeichnet, dass** der Wandler (3) in seinem mittleren Teil einen zweiten Ausgang (16) für das Kühlfluid aufweist, wobei die beiden Ausgänge vorzugsweise symmetrisch auf beiden Seiten der Schallausbreitungsachse der Sendeseite (4) angeordnet sind.

12. Ultraschallsonde nach Anspruch 11, **dadurch gekennzeichnet, dass** der Wandler (3) in seinem mittleren Teil einen Ausschnitt aufweist, der eine Aufnahme (6) für eine bildgebende Sonde (8) begrenzt, auf deren beiden Seiten sich die Ausgänge (16) für das Kühlfluid befinden.

13. Ultraschallsonde nach einem der Ansprüche 2 bis 12, **dadurch gekennzeichnet, dass** die Sonde eine Leitung (15) zum Zuführen von Fluid aufweist, die über den Eingang in die Kühlkammer (11) mündet, wobei diese Leitung eine Vorrichtung zum Steuern der Geschwindigkeit des Fluids aufweist.

14. Ultraschallsonde nach Anspruch 13, **dadurch gekennzeichnet, dass** sie einen Temperatursensor umfasst, der dazu ausgelegt ist, die Temperatur der Rückseite des Wandlers (3) zu messen, wobei dieser Temperatursensor mit der Vorrichtung zur Steuerung der Geschwindigkeit des Fluids verbunden ist, die ermöglicht, die Eintrittsgeschwindigkeit des Fluids in die Kühlkammer in Abhängigkeit von der Temperaturmessung zu steuern.

15. Ultraschallsonde nach einem der Ansprüche 2 bis 14, **dadurch gekennzeichnet, dass** die röhrenförmige Leitung (18) eine Länge besitzt, die größer als oder gleich entweder ihr(em) Durchmesser bei einer Leitung mit kreisförmigem Querschnitt oder der/dem kleinste(n) Durchmesser bei einer Leitung mit länglichem Querschnitt oder die/der kleinste(n) Breite bei einer Leitung mit rechteckigem Querschnitt ist.

## Claims

1. A method for cooling a transducer **(3)** of a probe for generating ultrasonic waves, the transducer **(3)** exhibiting, at the front, a face **(4)** for emitting ultrasonic waves with an axis of acoustic propagation **(A),** and at the rear, a rear face **(5),** at least the emitting face **(3)** partially delimiting a cooling chamber **(11)** wherein a cooling liquid is supplied through a tubular duct **(18)** extending upstream from an inlet **(14),** with a determined length along a longitudinal axis of extension **(X)** to channel the cooling liquid with a velocity vector, the cooling liquid circulating between at least the inlet **(14)** and at least an outlet **(16),** the inlet **(14)** being located at the periphery of the emitting face **(4)** whereas the outlet **(16)** is located in the central part of said emitting face, **characterised in that** it consists in orienting the tubular duct **(18)** in such a way that its longitudinal axis of extension **(X)** and the velocity vector which it carries have a tangential component established along a tangential direction substantially orthogonal to a plane **(P)** containing the acoustic propagation axis **(A)** and the inlet **(14),** and such that the ratio of the tangential component of the velocity vector on the centripetal component of the velocity vector is greater than 1, creating between the inlet **(14)** and the outlet **(16),** a circulation of the cooling fluid according to a swirling inside the cooling chamber around the axis of acoustic propagation **(A)** of the emitting face of the transducer.

2. An ultrasonic wave generating probe, including a transducer **(3)** exhibiting, at the front, a face **(4)** for emitting ultrasonic waves with an axis of acoustic propagation **(A)** for propagating ultrasonic waves, and at the rear, a rear face **(5),** at least the emitting face **(4)** partially delimiting a cooling chamber **(11)** wherein a cooling liquid is brought, through a tubular duct, to circulate between at least an inlet **(14)** located at the periphery of the emitting face **(4)** and at least an outlet **(16)** located in the central part of said emitting face, the tubular duct extending upstream of the inlet, with a length determined according to a longitudinal extension axis **(X)** to channel the cooling fluid with a velocity vector, **characterised in that** the tubular duct **(18)** is oriented such that its longitudinal extension axis **(X)** and the velocity vector which it carries have a tangential component establishing itself according to a tangential direction substantially orthogonal to a plane **(P)** containing the axis of acoustic propagation **(A)** and the inlet **(14),** and such that the ratio of the tangential component of the velocity vector on the centripetal component of the velocity vector is greater than 1, for guiding the fluid with a view to creating a circulation of cooling fluid according to a swirling inside the cooling chamber **(11).**

3. The ultrasonic probe according toclaim 2, **characterised in that** it includes at the inlet, at least one wall **(21, 2b)** for deflecting the fluid arranged facing the outlet of the tubular duct to orient the fluid according to the tangential direction.

4. The ultrasonic probe according to claim 3, **characterised in that** the emitting face **(4)** includes a peripheral edge **(4a)** located in a plane opening into an outlet, the tubular duct **(18)** for guiding the cooling fluid, a fluid deflecting wall **(21, 2b)** being arranged facing the outlet of the tubular duct.

5. The ultrasonic probe according to claim 4, **characterised in that** the peripheral edge **(4a)** of the emitting face **(4)** is bordered at its periphery by a peripheral channel **(28)** communicating with at least a tubular duct **(18)** for supplying cooling fluid, this channel **(28)** being provided with at least one centripetal opening for communicating with the cooling chamber.

6. The ultrasonic probe according to claim 5, **characterised in that** it includes several centripetal communication openings each delimited between two neighbouring wings **(29)** for diverting the cooling fluid, with a curving profile varying from one tangent direction to a quasi-radial direction.

7. The ultrasonic probe according to claim 2, **characterised in that** the probe **(1)** includes a tubular duct **(18)** for supplying cooling fluid and opening into the cooling chamber **(11)** and exhibiting a straight passage section whereof the normal is confounded with the tangential direction.

8. The ultrasonic probe according to any of claims 2 to 7, **characterised in that** the cooling chamber **(11)** includes a series of inlets **(14)** distributed at the periphery of the emitting face, each one exhibiting at least a tubular duct **(18)** for the fluid, oriented according to a direction for creating together a swirling circulation of the cooling fluid inside the cooling chamber **(11).**

9. The ultrasonic probe according to any of claims 2 to 8, **characterised in that** the emitting face **(4)** exhibits a focusing geometry.

10. The ultrasonic probe according to claim 9, **characterised in that** the emitting face **(4)** is truncated symmetrically with respect to an axis of acoustic propagation.

11. The ultrasonic probe according to any of claims 2 to 10, **characterised in that** the transducer **(3)** includes in its central part, a second outlet **(16)** for the cooling fluid, the two outlets preferably being, arranged symmetrically on either side of the axis of acoustic propagation of the emitting face **(4).**

12. The ultrasonic probe according to claim 11, **characterised in that** the transducer **(3)** includes in its central part, a cutout delimiting a housing **(6)** for an imaging probe **(8)** on either side of which are located the outlets **(16)** for the cooling fluid.

13. The ultrasonic probe according to any of claims 2 to 12, **characterised in that** the probe includes a fluid supply duct **(15)** opening by the inlet in the cooling chamber **(11),** this duct including a device for controlling fluid velocity.

14. The ultrasonic probe according to claim 13, **characterised in that** it includes a temperature sensor adapted for measuring the temperature of the rear face of the transducer **(3),** this temperature sensor being connected to the device for controlling the fluid velocity allowing to control the inlet velocity of the fluid in the cooling chamber according to the temperature measurement.

15. The ultrasonic probe according to any of claims 2 to 14, **characterised in that** the tubular duct **(18)** exhibits a length greater than or equal to either its diameter for a duct of circular section, or the smallest diameter for a duct of oblong section or the smallest width for a duct of rectangular section.
